# EUROPEAN PATENT SPECIFICATION

(11) **EP 1 578 327 B1**
(45) Date of publication and mention of the grant of the patent: **04.08.2010**
(21) Application number: 03754826.0
(22) Date of filing: 18.09.2003
(51) Int. Cl.: A61F 13/15

(54) **ABSORBENT ARTICLE WITH IMPROVED LEAK GUARDS**
ABSORBIERENDE ARTIKEL MIT VERBESSERTEM AUSLAUFSCHUTZ
ARTICLE ABSORBANT A PROTECTIONS CONTRE LES FUITES AMELIOREES

(30) Priority: 30.12.2002 US 331956
(43) Date of publication of application: 28.09.2005
(73) Proprietor: KIMBERLY-CLARK WORLDWIDE, INC., Neenah, WI 54956 (US)
(72) Inventor: OLSON, Christopher, P., Neenah, WI 54956 (US); STEVENS, Robert, A., Menasha, WI 54952 (US); FRANKE, Mark, S., Neenah, WI 54956 (US)
(74) Representative: Davies, Christopher Robert
(86) International application number: PCT/US2003/029843
(87) International publication number: WO 2004/060239

(56) References cited:
- EP-A- 0 670 154
- US-A- 5 695 849
- US-A- 5 899 894
- US-A- 5 952 252

## Description

### TECHNICAL FIELD OF THE INVENTION

The present invention relates generally to the field of absorbent articles and garments, such as children's training pants, disposable diapers, incontinence articles, and the like, which incorporate leak guards or containment flaps along their side edges.

### BACKGROUND

Many types of consumer products such as disposable diapers, training pants, feminine care articles, incontinence articles, and the like, utilize an absorbent pad structure for absorbing and wicking away bodily fluids. The absorbent pads are conventionally formed from an absorbent web disposed between a liquid pervious bodyside liner and a liquid impermeable outer cover. Such absorbent articles may include an elasticized waistband and leg cuffs to help reduce the leakage of body exudates. Some conventional absorbent articles have also included leak guards (also known in the art as "containment flaps") at the leg or waist sections of the article to further reduce the occurrence of leaks. These guards may be "elasticized" along their free longitudinal edge by incorporating elastic strips or strands along the free edge.

Conventional pant-like absorbent structures, such as a child's training pant, have also incorporated lateral leak guards alongside of the absorbent structure generally from the front waist band to the back waist band. Reference is made, for example, to the HUGGIES® PULL-UPS® disposable training pants from Kimberly-Clark Corporation of Neenah, Wisconsin. The use of containment flaps in training pants is also described, for example, in WO 00/35395 and U.S. Pat. No. 6,231,557 B1.

With typical training pant and disposable diaper configurations, the lateral containment flaps are formed of non-stretch or non-elastomeric materials. For example, a conventional leak guard used in the HUGGIES® PULL-UPS® disposable training pants is a laminate of a non-stretch polyethylene film and non-stretch spunbond web. However, with certain absorbent article configurations, the non-stretch leak guards may detract from the performance or properties of other stretch materials or components, such as stretch waistbands, stretch outer covers, stretch bodyside liners, and stretch absorbent structures. For example, if the non-stretch leak guards are attached (e.g., bonded or adhered) to a stretchable component such as an elastic waistband or elastomeric bodyside liner, the ability of such component to stretch as designed may be compromised. A prior art article, having the features of the preamble of claim 1, is shown in EP-0670154.

The present Invention provides an improved configuration for absorbent article leak guards that is compatible with other elastomeric or stretchable components in the article.

### SUMMARY OF THE INVENTION

According to the present invention, there is provided an absorbent article as claimed in claim 1.

Objects and advantages of the invention will be set forth in part in the following description, or may be obvious from the description, or may be learned through practice of the invention.

In general, the present invention provides an improved absorbent article having stretchable or elastomeric leak guards that cooperate with other stretchable components of the article while protecting against leakage from the sides of the absorbent structure contained in the article. A particularly useful embodiment of the invention is a child's training pant. However, other absorbent articles such as incontinence devices, disposable diapers, diaper pants, feminine hygiene products, swim pants, and the like, are also within the scope of the invention.

A pant-like absorbent article includes a chassis defining a front waist region at a first longitudinal end, a back waist region at an opposite longitudinal end, and a crotch region extending longitudinally between said front and back waist regions. The chassis may be defined generally entirely of elastomeric materials, or may only have particular regions or portions made up of elastomeric materials. It should be appreciated that the materials used in the article are not a limiting factor, and that any combination of known materials and compositions may be used in the manufacture of articles according to the invention.

In a typical training pant configuration, laterally extending panel portions are defined at opposed lateral sides of one or both of the front and back waist regions. These panel portions extend around the front and back waist of a wearer and meet at side seams of the article. The side panel portions may also define laterally inwardly directed leg contours. When the chassis is formed into the pant-like article, the panel portions are joined at the side seams and the leg contours define leg openings in conjunction with the crotch region of the article. This type of configuration is well known in the art.

In a desirable embodiment, at least the front and back panel portions of the chassis waist regions are elastomeric and thus stretchable. For example, elastomeric side panels may be attached to a generally non-elastomeric central portion of the chassis. In an alternate embodiment, essentially the entire chassis may be formed of elastomeric materials and be stretchable.

The article includes an absorbent body structure having a central portion that extends longitudinally along said crotch region generally from the front waist region to the back waist region. The absorbent body structure may have any desired shape depending on the type of absorbent article. For example, the absorbent body structure may be rectangular, T-shape, hour-glass shape, dogbone shape, etc. The absorbent body structure may include any combination of conventional absorbent materials used in absorbent articles. In one embodiment, the absorbent body structure may be generally elastomeric, and in another embodiment the structure may be generally non-elastomeric.

The absorbent article may include various types of side seam configurations. For example, the panel portions may be bonded at the side seams such that the wearer must pull the article on, much like underwear. These bonded seams may also be tearable such that the article may be removed from the wearer by separating or tearing at or along the seams and removing the article, much like a diaper. In an alternate embodiment, the side seams are releasable and re-attachable. For example, a hook-and-loop or other type of re-attachable system may be used along the side seams.

Longitudinally extending leak guards or containment flaps are provided on the body-facing side of the chassis. Each guard has longitudinal ends attached to the chassis generally at the waistband regions. The guards have a free laterally inward side that, in certain embodiments, may be elasticized in the longitudinal direction, for example by incorporating elastic strands along the free lateral side. Each guard has a laterally outward side attached to the chassis, for example to the bodyside liner, along its longitudinal dimension. The outward side is typically attached to the chassis outboard of the absorbent body structure. With conventional articles, the leak guards are generally elongated strips having parallel sides. However, this is not a limitation of the invention. Depending on their width (in the transverse direction), the guards may also be contoured to accommodate the leg openings. The leak guards desirably but not necessarily include a liquid impermeable or resistant material and define a containment pocket along the lateral sides of the absorbent body structure.

The leak guards are formed substantially of an elastomeric material or elastomeric composite material so as to be stretchable in the transverse direction. In this manner, regardless of the points of attachment of the guards laterally or at their respective longitudinal ends, the guards will not detract from the desired stretchable characteristics of another component of the article. For example, it is desirable to attach the longitudinal ends of the leak guards to the chassis at its elastic waistband structures. Non-elastomeric leak guards tend to inhibit or degrade the stretchability of the waistbands, which can lead to performance and comfort issues.

For performance, comfort, and wider body size range considerations, it may be desired to utilize more and more elastomeric materials in the construction of absorbent articles. For example, stretchable outer covers, stretchable bodyside liners, and stretch absorbents are finding particular usefulness. Elastomeric leak guards may be used to enhance the stretch properties of such components.

Aspects of the invention will be described below in greater detail with reference to embodiments shown in the figures.

### BRIEF DESCRIPTION OF THE FIGURES

Figure 1 is a perspective view of an embodiment of an absorbent article, in this case a child's training pant, in accordance with the invention.
Figure 2 is a simplified bodyside plan view of an absorbent article with all elastic components being in a tensioned state.
Figure 2A is a simplified bodyside plan view of an alternate embodiment of an absorbent article with all elastic components being in a tension state.
Figure 3 is a schematic cross-sectional view of the chassis taken along the lines indicated in Fig. 2.
Figure 3A is a schematic cross-sectional view of an alternate embodiment of an absorbent article according to the invention.

### DETAILED DESCRIPTION

The invention will now be described in detail with reference to particular embodiments thereof. The embodiments are provided by way of explanation of the invention, and are not meant as a limitation of the Invention. For example, features described or illustrated as part of one embodiment may be used with another embodiment to yield still a further embodiment. It is intended that the present invention include these and other modifications and variations as failing within the scope of the invention.

Within the context of the present description, the following terms may have the following meanings:
"Attached" refers to the bonding, joining, adhering, connecting, attaching, or the like, of two elements. Two elements will be considered to be attached together when they are bonded directly to one another or indirectly to one another, such as when each is directly attached to an intermediate element.
"Elastomeric" refers to a material or composite which can be elongated by at least 25% of its relaxed length and which will recover, upon release of the applied force, at least 10% of its elongation. It is generally preferred that the elastomeric material or composite be capable of being elongated by at least 100%, more preferably by at least 300%, of it relaxed length and recover at least 50% of its elongation. An elastomeric material is thus stretchable and "stretchable" and "elastomeric," may be used interchangeably.
"Elastic" or "Elasticized" means that property of a material or composite by virtue of which it tends to recover towards its original size and shape after removal of a force causing a deformation.
"Neck-bonded" laminate refers to a composite material having an elastic member that is bonded to a non-elastic member while the noun-elastomeric member is extended in the machine direction creating a necked material that is elastic in the cross-direction. Examples of neck-bonded laminates are disclosed in U.S. Pat. Nos. 4,965,122; 4,981,747; 5,226,992; and 5,336,545.
"Stretch-bonded" laminate refers to a composite material having at least two layers in which one layer is a gatherable layer and the other layer is an elastic layer. The layers are joined together when the elastic layer is in an extended condition so that upon relaxing the layers, the gatherable layer is gathered. For example, one elastic member can be bonded to another member while the elastic member is extended at least about 25% of its relaxed length. Such a multiplayer composite elastic material may be stretched until the non-elastic layer is fully extended. Examples of stretch-bonded laminates are disclosed, for example, in U.S. Patent Nos. 4,720,415, 4,789,699, 4781,968, 4,657,802, and 4,655,760.
"Member" when used in the singular can refer to a single element or a plurality of elements.

Various aspects and embodiments of the Invention will be described in the context of a disposable absorbent article, such as a child's training pant, It is, however, readily apparent that the present invention could also be employed with other articles, including disposable diapers, feminine care articles, incontinence garments, diaper pants, swim pants, and the like. Typically, the disposable articles are intended for limited use and are not intended to be laundered or otherwise cleaned for reuse. A child's training pant, for example, is discarded after it has become soiled by the wearer. In its various aspects, the invention can provide a distinctive absorbent article which exhibits desirable physical properties, such as softness, flexibility, conformance, trim appearance, reduced gapping and reduced leakage, particularly from around the sides of the article. The article can include an absorbent body structure which exhibits desirable physical properties, such as improved softness, flexibility, durability, conformance and stretchability. As a result, the absorbent structures and articles of the invention can provide increased strength, improved fit, reduced leakage, and reduced clumping, bunching or sagging during use.

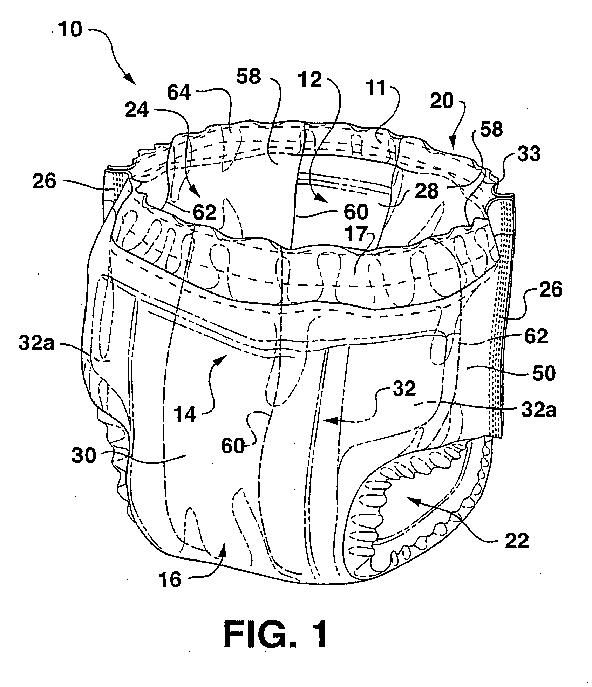

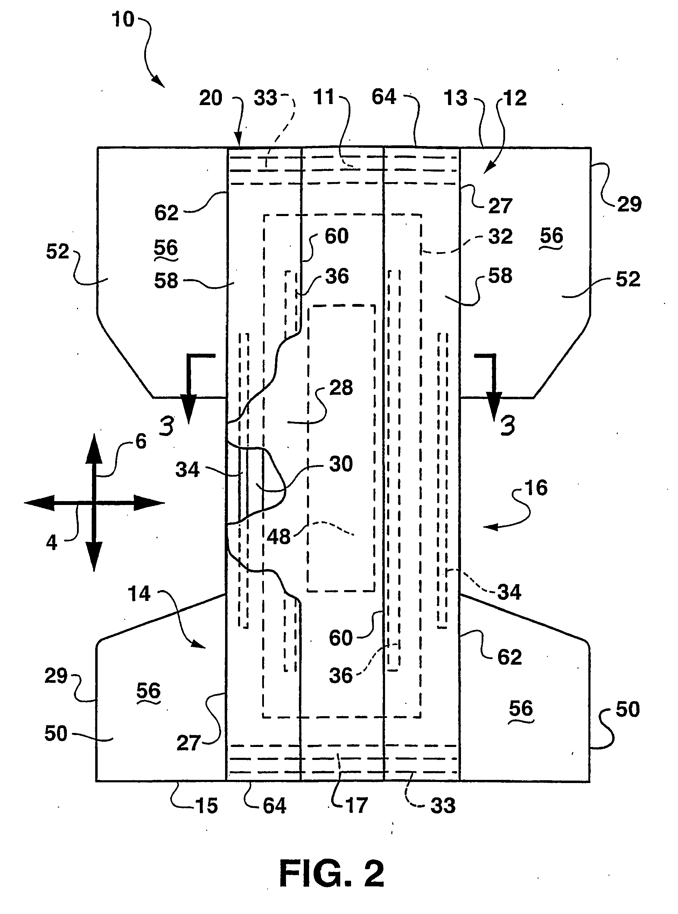

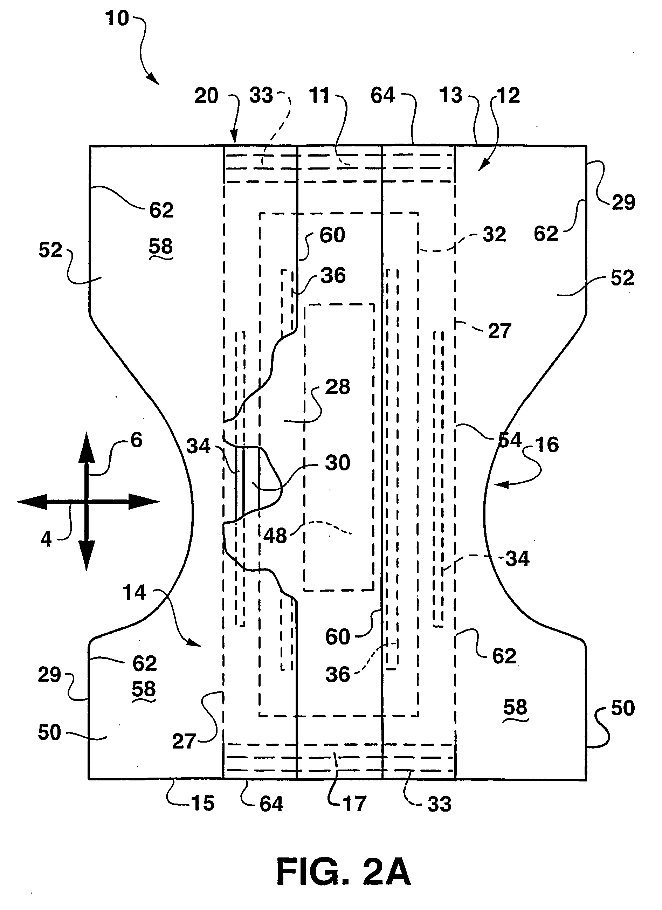

With reference to the figures, an article, such as the representatively shown child's training pant 10, includes a body or chassis 20 having a lengthwise, longitudinal direction 6, a lateral, transverse cross-direction 4, a front waist region 14, a back waist region 12, and an intermediate crotch region 16 interconnecting the front and back waist regions. The waist regions 12 and 14 comprise those portions of the article 10 which when worn, wholly or partially cover or encircle the waist or mid-lower torso of the wearer. The front 14 and back 12 waist regions includes elastic front and back waistband portions 17, 11. In the embodiment of Fig. 1, the elastic waistband portions 11,17 are generally continuous around the waist opening 24 of the article. In the embodiment of Fig. 2, the waistband portions 11,17 extend only partially across their respective waist regions. The intermediate crotch region 16 lies between and interconnects the waist regions 14 and 12, and comprises that portion of the article 10 which, when worn, is positioned between the legs of the wearer and covers the lower torso of the wearer. Thus, the intermediate crotch region 16 is an area where repeated fluid surges typically occur in the training pant or other disposable absorbent article.

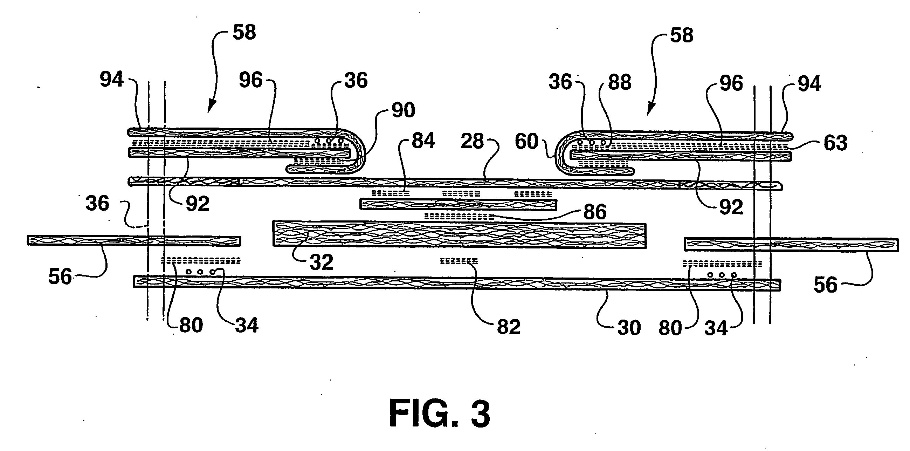

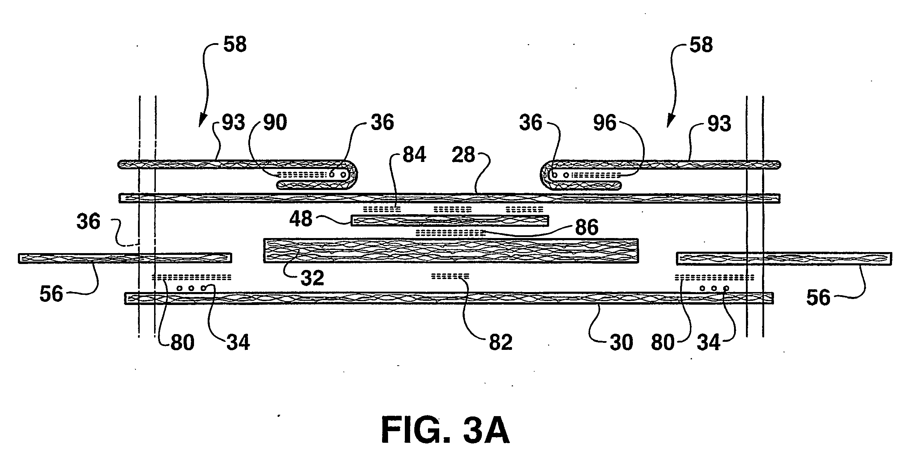

The article 10 includes a substantially liquid-impermeable outer cover member 30, a liquid-permeable bodyside liner 28, and an absorbent body structure 32 sandwiched between the outer cover member 30 and the bodyside liner layer 28. The absorbent body structure may be secured to the outer cover member 30 by an adhesive 82, and secured to the bodyside liner 28 by an adhesive 84, as represented in Fig. 3. In certain configurations, the outer cover member 30 and absorbent body structure 32 may be stretchable, particularly in the lateral or transverse direction 24. For example, the outer cover member 30 may be made substantially of an elastomeric material, and the absorbent body structure may include an elastomeric absorbent material. In certain embodiments, the bodyside liner 28 is also stretchable. For example, the bodyside liner 28 may be made of an elastomeric material.

In the illustrated embodiment of the article 10, the chassis 20 includes laterally extending front panel portions 50 and back panel portions 52. This configuration is common for training pants. In the embodiment of Fig. 1, the panel portions are an extension of the chassis 20 and may be, for example, extensions of the outer cover member 30, bodyside liner 28, or both. This configuration may be desirable particularly if the chassis 20, including the central structure 16, is stretchable in the transverse direction 4, particularly across the front and back waist regions 14, 12. For example, the chassis may include an elastomeric cover member 30, elastomeric bodyside liner 28, and any combination of other elastomeric components that in combination render a stretchable unitary chassis. In an alternate embodiment as illustrated in Fig. 2, the panel portions 50, 52 are defined by generally elastomeric side panels 56 that are attached to lateral sides 54 of the chassis 20, for example along seam lines 27 with an adhesive 80. In this embodiment, the components of the central structure of the chassis 20 may or may not be elastomeric.

Referring to Fig. 1, the absorbent body structure 32 may include laterally extending wing portions 32a that extend laterally into the panel portions 50, 52. These wing portions may be at the front waist region, the back walst region, or both of the front and back waist regions.

The training pant 10 may be of a style and configuration wherein the front and back panel portions 50, 52 have lateral sides 29 that are brought together upon folding the chassis to form a pant structure having the waist opening 24 and leg openings 22. The lateral sides 29 are bonded In a known manner so as to define side seams 26 of the pant structure. With this type of configuration, the pant 10 Is pulled on by the wearer in a manner similar to underwear. Desirably, these seams 26 may be separable or tearable so that the pant 10 may be removed from the wearer by tearing at or along the seams 26 and removing the article in a manner similar to a diaper. In an alternate embodiment, the front and back panel portions 50, 52 may be separable and re-attachable at the side seams 26. A fastening system, such as a hook-and-loop system, may be used to Interconnect the first waist region 12 with the second waist region 14 to define the pant structure and hold the article on a wearer. Additional suitable releasable fastening systems are described in U.S. Pat. No, 6,231,557 B1 and the International Application WO 00/35395.

The article 10 also incorporates longitudinally extending leak guards 58 disposed over the bodyside liner 28 in the body facing direction. The guards 58 have longitudinal ends 64 that are attached to the chassis 20 at the longitudinal ends 13, 15 thereof. For example, the guards 58 may extend over and be attached to the waistband portions 11, 17, for example by ultrasonic bonding, The guards 58 have an outboard lateral side 62 that is attached to the chassis desirably outboard of the underlying absorbent body structure 32, as shown by the dashed lines in Fig. 1. Examples of conventional leak guards may be found, for example, In the HUGGIES® PULL-UPS® disposable training pants. Referring to Fig. 1, the lateral sides 62 (dashed lines) may also extend to the side seams 26 and be attached in a common bonding process with the side seams 26. In an alternate embodiment, the lateral side 62 may not be outboard of the absorbent body structure, but extends over a portion of the absorbent body structure 32. Referring to Fig. 3, the guards 58 may be attached, for example, along a generally continuous ultrasonic bond line 63. The guards 58 have a laterally inboard "free" side 60 such that the guards essentially define a containment pocket along the lateral sides of the absorbent structure 32. The leak guards 58 according to the present invention will be described in greater detail below.

Fig. 2 and Fig. 2A show a body facing plan views of alternative representative articles 10 in a generally flat-out, uncontracted state (i.e., with substantially all elastic induced gathering and contraction removed). In Fig. 2, the elastomeric leak guards 58 have a laterally outboard side 62 that is bonded along lines 27. In Fig. 2A, the laterally outboard sides 62 extend to the lateral sides 29 of the chassis and would be attached at the side seams 26. In this embodiment, the leak guards 58 may be coextensive with the liner 28 and/or outer cover member 30, all of which may be elastomeric, such that the outboard sides 62 are coextensive with the chassis lateral sides 29. In an alternate embodiment, the portions of the leak guards 58 in the side panel regions 50, 52 may actually be the sole material defining the side panels. In other words, the liner 28 and outer cover member 30 may be generally attached along the central portion of the chassis 20 along the lines 27 (dashed line in Fig. 2A) with the leak guards being the only material defining the panels 50, 52 and being joined at side seams 26. In yet another alternative embodiment, the leak guards may be coextensive with attached elastomeric side panels 56.

As mentioned, the article 10 will typically include a porous, liquid permeable bodyside liner 28; a substantially liquid impermeable outer cover member 30; and an absorbent body structure 32 positioned and attached between the outer cover member and bodyside liner. In certain embodiments, a surge layer 48 may be optionally located adjacent the absorbent structure and attached by way of adhesive 84 to the liner 28 (Fig. 3). Elastomeric gathering members, such as leg elastics 34 and waist elastics 33 may be provided, as is well known in the art. The liner 28, outer cover 30, absorbent structure 32, surge layer 48, and elastic members 34 and 32 may be assembled together Into a variety of well-known absorbent article configurations,

The leg elastic members 34 may be located in the lateral side margins of the chassis 20, particularly along the lateral sides of the crotch region 16, and are configured to draw and hold the chassis 20 against the legs of the wearer. The elastic members 34 are secured to the chassis 20 in an elastically contracted state so that in a normal under strain condition, the elastic members 34 effectively contract against the chassis. An adhesive 80 (Figs. 3 and 3A) attaches the leg elastic members 34 to the outer cover 30 and one or both of the side panel 56 and leak guard 58. The use of elastic leg members in absorbent articles such as disposable diapers and training pants Is widely known and understood In the art.

The use of elastic waistbands is also widely known and used in the art. In the illustrated embodiment of Fig. 1, the waist elastics 33 are provided generally across the entirety of the front and back waistbands 17, 11. In alternate embodiments, the waist elastics 33 may extend only partially across the front and back waistbands, as represented In Fig. 2. The waist elastics 33 may be composed of any suitable elastomeric material, such as an elastomeric film, an elastic foam, multiple elastic strands, an elastomeric fabric, and the like. Embodiments of waistband structures that may be utilized with articles 10 according to the invention are also described in U.S. Patent Nos. 5,601,547; 6,358,350 B1; 6,336,921 B1; and 5,711,832.

The liner 28 and outer cover member 30 may be generally coextensive, and may have length and width dimensions which are generally larger than and extend beyond the corresponding dimensions of the absorbent structure 32 to provide for the corresponding side and end margins. Optionally, the bodyside liner 28 and outer cover member 30 may not be coextensive. The outer cover member 30 may be composed of a liquid permeable material, but desirably comprises a material which is configured to be substantially impermeable to liquids. For example, a typical outer cover can be manufactured from a thin plastic film, a composite laminate, or other flexible, substantially liquid-impermeable material. As used in the present specification, the term "flexible" refers to materials which are compliant and which will readily conform to the general shape and contours of the wearer's body.

Alternative constructions of the outer cover member 30 may comprise a woven or non-woven fibrous web layer which has been totally or partially constructed or treated to impart the desired levels of liquid impermeability to selected regions that are adjacent or proximate the absorbent body. For example, the outer cover may include a gas-permeable, nonwoven fabric layer laminated to a polymer film layer which may or may not be gas-permeable. Other examples of fibrous, cloth-like outer cover materials can comprise a stretch thinned or stretch thermal laminate material. Although the outer cover 30 typically provides the outermost layer of the article, optionally the article may include a separate outer cover component member which is additional to the outer cover member.

In a particular embodiment, the outer cover 30 may be formed substantially from an elastomeric material and is thus stretchable. In this embodiment, the outer cover 30 may define the entire front and back waist regions 14, 12, including the front and back panel portions 50, 52. The outer cover 30 may, for example, be composed of a single layer, multiple layers, laminates, spunbond fabrics, films, meltblown fabrics, elastic netting, microporous web, bonded carded webs or foams comprised of elastomeric or polymeric materials. Elastomeric nonwoven laminate webs may include a nonwoven material joined to one or more gatherable nonwoven webs, films, or foams. Stretch Bonded Laminates (SBL) and Neck Bonded Laminates (NBL) are examples of elastomeric composites. Nonwoven fabrics are any web of material which has been formed without the use of textile weaving processes which produce a structure of individual fibers which are interwoven in an identifiable repeating manner. Examples of suitable materials are Spunbond-Meltblown fabrics, Spunbond-Meltblown-Spunbond fabrics, Spunbond fabrics, or laminates of such fabrics with films, foams, or other nonwoven webs. Elastomeric materials may include cast or blown films, foams, or meltblown fabrics composed of polyethylene, polypropylene, or polyolefin copolymers, as well as combinations thereof. The elastomeric materials may include polyether block amides such as PEBAX® elastomer (available from AtoChem located in Philadelphia, Pa.), HYTREL® elastomeric polyester (available from E. I. DuPont de Nemours located in Wilmington, Del.), KRATON® elastomer (available from Shell Chemical Company located in Houston, Tex.), or strands of LYCRA® elastomer (available from E.I. DuPont de Nemous located in Wilmington, Del.), or the like, as well as combinations thereof. The outer cover 30 may include materials that have elastomeric properties through a mechanical process, printing process, heating process, or chemical treatment. For examples such materials may be apertured, creped, neck-stretched, heat activated, embossed, and micro-strained; and may be in the form of films, webs, and laminates.

In an alternate embodiment, the chassis 20 may be substantially non-elastomeric along the crotch region 16. In this embodiment, the outer cover 30 may be made of a generally non-elastomeric material and essentially covers only the central structure of the chassis 20. As discussed, elastomeric side panels 56 may provide a desired degree of stretchability to the chassis 20, particularly around the waist and sides of the wearer. A structure of this type is known, for example, from the HUGGIES® PULL-UPS® disposable training pants. The pair of transversely opposed side panels 56 may be permanently bonded to the lateral sides of the chassis 16 at bond lines 63 (Fig. 3) using attachment means known to those skilled in the art, such as adhesive, thermal or ultrasonic bonding. Particular examples of suitable constructions for securing a pair of elastically stretchable members to the lateral, side portions of an article to extend laterally outward beyond the laterally opposed side regions of the outer cover and liner components of an article can be found in U.S. Pat. No. 4,938,753. The lateral outboard sides of the side panels 56 may then be permanently or releasably attached along side seams 26 to define a pant structure. These bonded side seams may be tearable as discussed above. Alternately, the side panels may be releasably attachable along the side seams 26 using any type of suitable releasable fastener system, as discussed above.

Suitable elastic materials for the side panels 56, as well as a described process of incorporating elastic side panels into a training pant, are described, for example, in the following U.S. Patents: 4,940,464; 5,224,405; 5.104.116: 5,046,272; and WO 01/88245. In particular embodiments, the elastic material comprises a stretch-thermal laminate (STL), a neck-bonded laminate (NBL), a reversibly necked laminate, or a stretch-bonded laminate (SBL) material. Methods of making such materials are described, for example, In U.S. Patent Nos. 4,663,220; 5,226,992; and the EP Application 0 217 032.

The bodyside liner 28 presents a body-facing surface which is compliant, soft-feeling, and non-irritating to the wearer skin. Further, the bodyside liner 28 can be less hydrophilic than the absorbent body 32, and is sufficiently porous to be liquid permeable, permitting liquid to readily penetrate through its thickness to reach the absorbent body. A suitable bodyside liner layer 28 may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. The bodyside liner layer 28 is typically employed to help isolate the wearer's skin from liquids held in absorbent body 32.

Various woven and nonwoven fabrics can be used for bodyside liner 28. For example, the bodyside liner may include a meltblown web, a spunbonded web, or a bonded-carded-web composed of the desired fibers. The various fabrics can be composed of natural fibers, synthetic fibers or combinations thereof. In particular aspects, the bodyside liner 28 may be comprised of polymer fibers, networks, laminates, liquid permeable films, cellulosic fibers, rayon, water swellable gels, and elastomeric materials, as well as combinations thereof. Suitable materials for the bodyside liner can include meltblown webs, airlaid webs, spunbond webs, or bonded-carded webs of synthetic continuous or discrete polymer fibers and/or natural fibers, a pattern bonded spunbonded web, airlaid web, or bonded carded web, as well as combinations thereof. Suitable polymers can include polypropylene, polyethylene, polyester, and bicomponent materials composed of these polyolefins.

The bodyside liner fabrics may be composed of a substantially hydrophobic material, and the hydrophobic material may optionally be treated with a surfactant or otherwise processed to impart a desired level of wettability and hydrophilicity. In a particular embodiment of the invention, the bodyside liner 28 can be a nonwoven, spunbond polypropylene fabric which is necked approximately 60%. Strands of about KRATON® G2760 elastomer material may be adhered to the necked spunbond material. The fabric can be surface treated with an operative amount of surfactant, such as about 0.6% AHCOVEL Base N62 surfactant, available from ICI Americas, a business having offices located in Wilmington, Del. The surfactant can be applied by any conventional means, such as spraying, printing, brush coating or the like.

In particular embodiments wherein it is desired that the bodyside liner layer 28 be stretchable, suitable elastomeric materials can include elastic strands, LYCRA® elastics, elastic films, cast or blown; nonwoven elastic webs, meltblown or spunbond elastomeric fibrous webs, as well as combinations thereof. Examples of elastomeric materials include KRATON® elastomers, HYTREL® elastomers, ESTANE® elastomeric polyurethanes (available from B.F. Goodrich and Company located in Cleveland, Ohio), or PEBAX® elastomers. The bodyside liner may include blends or laminates of fibers, scrim, webs, and films with perforations, apertures, creping, heat activation, embossing, micro-straining, chemically treatment, or the like, as well as combinations thereof.

The bodyside liner 28 and outer cover 30 are connected or otherwise associated together in an operable manner. As used herein, the term "associated" encompasses configurations in which the bodyside liner 28 is directly joined to the outer cover 30 by affixing the bodyside liner 28 directly to the outer cover 30, and configurations wherein the bodyside liner 28 is indirectly joined to the outer cover 30 by affixing the bodyside liner 28 to intermediate members which in turn are affixed to the outer cover 30. The bodyside liner 28 and outer cover 30 can, for example, be joined to each other in at least a portion of the training pant periphery by suitable attachment mechanisms (not shown) such as adhesive bonds, ultrasonic bonds, thermal bonds, pinning, stitching or any other attachment technique known in the art, as well as combinations thereof. For example, a uniform continuous layer of adhesive, a patterned layer of adhesive, a sprayed pattern of adhesive or an array of separate lines, swirls or spots of construction adhesive may be used to affix the bodyside liner 28 to the outer cover 30. It should be readily appreciated that the above-described attachment means may also be employed to suitably interconnect, assemble and/or affix together the various other component parts of the articles which are described herein.

The absorbent body structure 32 is positioned between the outer cover 30 and the bodyside liner 28. The absorbent body structure 32 can be any structure or combination of components which are generally compressible, conformable, non-irritating to a wearer's skin, and capable of absorbing and retaining liquids and certain body wastes. For example, the structure 32 may include an absorbent web material of cellulosic fibers (e.g., wood pulp fibers), other natural fibers, synthetic fibers, woven or nonwoven sheets, scrim netting or other stabilizing structures, superabsorbent material, binder materials, surfactants, selected hydrophobic materials, pigments, lotions, odor control agents or the like, as well as combinations thereof. In a particular embodiment, the absorbent web material is a matrix of cellulosic fluff and superabsorbent hydrogel-forming particles. The cellulosic fluff may comprise a blend of wood pulp fluff. One preferred type of fluff is identified with the trade designation CR 1654, available from U.S. Alliance of Childersburg, Alabama, USA, and is a bleached, highly absorbent sulfate wood pulp containing primarily soft wood fibers. The absorbent materials may be formed into a web structure by employing various conventional methods and techniques. For example, the absorbent web may be formed with a dry-forming technique, an air forming technique, a wet-forming technique, a foam-forming technique, or the like, as well as combinations thereof. Methods and apparatus for carrying out such techniques are well known in the art.

As a general rule, the superabsorbent material is present in the absorbent web in an amount of from about 0 to about 90 weight percent based on total weight of the web. The web may have a density within the range of about 0.10 to about 0.35 grams per cubic centimeter.

Superabsorbent materials are well known in the art and can be selected from natural, synthetic, and modified natural polymers and materials. The superabsorbent materials can be inorganic materials, such as silica gels, or organic compounds, such as crosslinked polymers. Typically, a suberabsorbent material is capable of absorbing at least about 15 times its weight in liquid, and desirably is capable of absorbing more than about 25 times its weight in liquid. Suitable superabsorbent materials are readily available from various suppliers. For example, Favor 880 superabsorbent is available from Stockhausen GmbH of Germany; and Drytech 2035 is available from Dow Chemical Company, of Midland Michigan, USA.

After being formed or cut into a desired shape, the absorbent web material may be wrapped or encompassed by a suitable tissue wrap that aids In maintaining the integrity and shape of the absorbent structure 32.

The absorbent web material may also be a coform material. The term "coform material" generally refers to composite materials comprising a mixture or stabilized matrix of thermoplastic fibers and a second non-thermoplastic material. As an example, coform materials may be made by a process in which at least one meitblown die head is arranged near a chute through which other materials are added to the web while it is forming. Such other materials may include, but are not limited to, fibrous organic materials such as woody or non-woody pulp such as cotton, rayon, recycled paper, pulp fluff and also superabsorbent particles, inorganic absorbent materials, treated polymeric staple fibers and the like. Any of a variety of synthetic polymers may be utilized as the melt-spun component of the coform material. For instance, in some embodiments, thermoplastic polymers can be utilized. Some examples of suitable thermoplastics that can be utilized include polyolefins, such as polyethylene, polypropylene, polybutylene and the like; polyamides; and polyesters. In one embodiment, the thermoplastic polymer is polypropylene. Some examples of such coform materials are disclosed in U.S. Patent Nos. 4,100,324 to Anderson, et al.; 5,284,703 to Everhart, et al.; and 5,350,624 to Georger, et al.

With particular embodiments of an absorbent article according to the invention, it may be desired that the absorbent body structure 32 is elastomeric. For example, in the embodiment as shown in Fig. 1 wherein generally the entire chassis 20 Is stretchable or elastomeric, it may be desired that the absorbent body structure 32 is also stretchable so as not to inhibit the stretchability of other components. For this purpose, the absorbent web material can include elastomeric fibers in an amount which is at least a minimum of about 2 wt %. The amount of elastomeric fibers can alternatively be at least about 3 wt %, and can optionally be at least about 5 wt % to provide improved performance. In addition, the amount of elastomeric fibers can be not more than about 60 wt %. Alternatively, the amount of elastomeric fibers can be not more than about 45 wt %, and optionally, can be not more than about 30 wt % to provide improved benefits. These values are important because they can provide the absorbent retention portion with desired levels of stretchability and structural stability without excessively degrading the physical properties or the liquid-management properties of the absorbent structure. An absorbent web material with an excessively low proportion of elastomeric fibers may be insufficiently stretchable, and a web material with an excessively high proportion of elastomeric fibers may exhibit an excessive degradation of its absorbency functionalities, such as poor intake, poor distribution, poor retention of liquid and/or an excessive tension force when stretched.

The absorbent body structure 32 may include an elastomeric coform absorbent web material. In particular aspects, the elastomeric coform material can have an overall coform basis weight which is at least a minimum of about 50 grams per square meter (g/m²). The coform basis weight can alternatively be at least about 100 g/m² and can optionally be at least about 200 g/m² to provide improved performance. In addition, the coform basis weight can be not more than about 1200 g². Alternatively, the coform basis weight can be not more than about 900 g/m², and optionally, can be not more than about 600 g/m² to provide improved benefits. These values are important because they can provide the absorbent body structure with desired stretchability and structural stability without excessively degrading the physical properties or the liquid-management functionalities of the absorbent body structure, Retention portions having excessively low proportions of elastomeric coform material may not be sufficiently stretchable. An absorbent web material having excessively large amounts of elastomeric coform materials can exhibit an excessive degradation of their absorbency functionalities, such as an excessive degradation of intake, distribution and/or retention properties.

Other examples of elastomeric absorbent structures are described in U.S. Patent No. 6,362,389 B1.

The absorbent web material utilized in the absorbent body structure 32 is also selected so that the individual absorbent body structure possesses a particular individual total absorbency depending on the intended article of use. For example, for infant care products, the total absorbency can be within the range of about 200-900 grams of 0.9 wt% saline, and can typically be about 500g of saline. For adult care products, the total absorbency can be within the range of about 400-2000 grams of saline, and can typically be about 1300g of saline. For feminine care products, the total absorbency can be within the range of about 7-50 grams of menstrual fluid, and can typically be within the range of about 30-40 g of menstrual fluid. '

The absorbent body structure 32 may also Indue a surge management layer 48 which helps to decelerate and diffuse surges or gushes of liquid that may be rapidly introduced into the absorbent body of the article. Desirably, the surge management layer can rapidly accept and temporarily hold the liquid prior to releasing the liquid into the storage or retention portions of the absorbent structure. The surge layer can be located below the bodyside liner layer 28. Alternatively, the surge layer may be located on the body facing surface of the bodyside liner 28. Examples of suitable surge management layers are described in U.S. Pat. No. 5,486,166; and U.S. Pat. No. 5.490,846. Other suitable surge management materials are described in U.S. Pat. No. 5,820,973.

As discussed, the leak guards 58 are desirably formed substantially of an elastomeric material or elastomeric composite material so as to be stretchable in the transverse direction. In this manner, regardless of the points of attachment of the guards laterally or at their respective longitudinal ends, the guards will not detract from the desired stretchable characteristics of another component of the article. The leak guards 58 are not limited to any particular type of elastomeric material, so long as such material is moisture resistant and preferably presents a cloth-like feel to the wearer. Suitable materials may be, for example, a neck-bonded laminate, stretch-bonded laminate, stretch-thermal laminate, or any combination of materials that provides moisture resistance and a desired degree of cloth-like feel to the wearer. A particularly suitable material is a neck-bonded laminate (NBL) of an elastic polyethylene film and neck stretched spunbond polypropylene. As represented in Fig. 3, the guards 58 may be a laminate composite of a liquid impervious elastic film 92 and the necked spunbond web 94. An adhesive 96 is used to laminate the materials together in combination with ultrasonic welding. Elastic strands 36 may be incorporated into the laminating process with an adhesive 88 to produce the type of structure shown In Fig. 3. An extension or "flap" of the necked spunbond web 94 may be folded over along the free side 60 of the guards 58 and attached to the underside of the elastic film 92 with an adhesive 90- It should be appreciated, however, that the invention is not limited to this structural configuration of leak guards 58. Suitable other constructions and arrangements for the leak guards 58 are generally known to those skilled in the art and examples are also described In U.S. Pat. No. 4,704,116.

An alternate leak guard configuration is shown in Fig. 3A. In this embodiment, the leak guards are formed from a single layer of material 93, for example any suitable generally elastomeric and liquid impervious or resistant material. The material 93 is folded under at the free edge 60 and bonded with, for example, and adhesive 90. Elastic strands may be incorporated into the fold.

In a particular embodiment, a suitable elastic film 92 is available from Huntsman Packaging and identified as XMAX-314.0. A suitable necked spunbond web is a 0.25 to 0.5 osy polypropylene necked down to about 50% to about 40% of its original width resulting in a final necked basis weight of approximately 0.55 to 1.1 osy. A suitable hot melt adhesive used for laminating the materials together is H2525A (RM7499) from Findley Adhesives, Inc. of Wauwatosa, Wisconsin, U.S.A.

It should be understood that resort may be had to various other embodiments, modifications, and equivalents to the embodiments of the Invention described herein which, after reading the description of the invention herein, may suggest themselves to those skilled in the art without departing from the scope and the present invention.

## Claims

1. An absorbent article (10), comprising:
a chassis (20) having a front waist region (14) at a first longitudinal end (15), a back waist region (12) at an opposite longitudinal end (13), and a crotch region (16) extending longitudinally between said front and back waist regions (14, 12);
an absorbent body structure (32) extending longitudinally generally from said front waist region (14) to said back waist region (12); and
longitudinally extending leak guards (58), each said leak guard (58) having a free laterally inward side (60) and a laterally outward side (62) attached to said chassis (20) so as to define respective containment pockets along opposite lateral sides of said absorbent body structure (32), wherein said chassis (20) includes stretchable waistbands (11,17) along at least a portion of said front and back waist regions (14,12), said leak guards (58) having longitudinal ends (64) attached to said chassis (20) coextensive with said stretchable waistbands (11,17).
**characterised in that:**
said leak guards (58) are elastomeric in a transverse direction (4) along their longitudinal length.

2. The absorbent article (10) as in claim 1, wherein said article (10) is a child's training pant.

3. The absorbent article (10) as in claim 1 or 2, wherein said chassis (20) includes a substantially elastomeric outer cover member (30), said laterally outward sides of said leak guards (58) attached to said elastomeric outer cover member (30).

4. The absorbent article (10) as in claim 1 or 2, wherein said chassis (20) includes a substantially elastomeric bodyside liner (28), said laterally outward sides (62) of said leak guards (58) attached to said elastomeric bodyside liner (28).

5. The absorbent article (10) as in claim 1 or 2, wherein said chassis (20) comprises a generally non-elastomeric structure, and further comprising elastomeric side panels (56) attached to lateral sides of said chassis (20), said laterally outward sides (62) of said leak guards (58) attached to said chassis (20) with said elastomeric side panels (56).

6. The absorbent article (10) as in claim 5, wherein said side panels (56) define laterally extending front and back panel portions (50,52) at opposed lateral sides of said front and back waist regions (14,12).

7. The absorbent article (10) as in claim 6, wherein said leak guards (58) have longitudinal ends (64) attached to said side panels (56) at said waist regions (14,12) of said chassis (20).

8. The absorbent article (10) as in any preceding claim, wherein said laterally inward sides (60) of said leak guards (58) are elasticized in the longitudinal direction (6).

9. The absorbent article (10) as in any preceding claim, wherein said leak guards (58) are formed of a liquid impermeable or liquid resistant neck-bonded-laminate material.

10. The absorbent article (10) as in claim 9, wherein said neck-bonded laminate material comprises a necked stretched polypropylene spunbond web laminated to an elastic film.

11. The absorbent article (10) as in any of claims 1 to 8, wherein said leak guards (58) are formed of a liquid impermeable stretch-bonded-laminate material.

12. The absorbent article (10) of claim 1, wherein:
said chassis (20) comprises a generally elastomeric absorbent chassis (20) comprising a bodyside liner (28), an outer cover (30) bonded to said bodyside liner (28), and an absorbent structure (32) disposed between said bodyside liner (28) and said outer cover (30);
said front and back waist regions (14,12) further comprise laterally extending side panel portions (56), said panel portions (56) defining leg contours with said crotch region (16) along opposite lateral sides of said chassis (20);
said leak guards (58) define said containment pockets along said leg contours and said side panel portions (56); and
upon forming said chassis (20) into a pant structure with front and back side panel portions (56) joined at side seams (26) of said article (10), said leak guards (58) provide leakage protection to a wearer generally completely around the front and back waist regions (14,12).

13. The absorbent article (10) as in claim 12, wherein said absorbent structure (32) further comprises a central longitudinally extending portion and elastomeric wing portions (32a) that extend laterally into said front and back side panel portions (56).

14. The absorbent article (10) as in claim 13, wherein said absorbent structure (32) includes an elastomeric absorbent material along said central portion and said wing portions (32a).

15. The absorbent article (10) as in claim 14, wherein said absorbent structure (32) is attached to said outer cover (30) along said central portion and said wing portions.

16. The absorbent article (10) in any of claims 12 to 15, wherein said absorbent structure (32) extends centrally along said crotch region (16) between said front and back waist regions (14,12).

17. The absorbent article (10) as in any of claims 12 to 16, wherein said article (10) is a child's training pant.

18. The absorbent article (10) as in any of claims 12 to 17, wherein said side seams (26) are bonded tearable seams.

19. The absorbent article (10) as in any of claims 12 to 17, wherein said side seams (26) are releasable and re-attachable.

20. The absorbent article (10) as in any of claims 12 to 19, wherein said leak guards (58) are made of an elastomeric material.

## Patentansprüche

1. Absorptionsfähiger Artikel (10), welcher umfasst:
einen Rahmen (20), der einen vorderen Taillenbereich (14) an einem ersten Längsende (15), einen hinteren Taillenbereich (12) an einem gegenüberliegenden Längsende (13) und einen Schrittbereich (16) aufweist, der sich längs zwischen dem vorderen und hinteren Taillenbereich (14, 12) erstreckt;
eine absorptionsfähige Körperstruktur (32), die sich längs generell von dem vorderen Taillenbereich (14) zu dem hinteren Taillenbereich (12) erstreckt; und
sich längs erstreckende Leckschutzvorrichtungen (58), wobei jede der Leckschutzvorrichtungen (58) eine freie seitliche Innenseite (60) und eine seitliche Außenseite (62) aufweist, die am Rahmen (20) befestigt ist, um so entsprechende Auffangtaschen entlang gegenüberliegenden seitlichen Seiten der absorptionsfähigen Körperstruktur (32) zu definieren, wobei der Rahmen (20) dehnbare Taillenbänder (11, 17) entlang zumindest eines Teils des vorderen und hinteren Taillenbereichs (14, 12) beinhaltet, wobei die Leckschutzvorrichtungen (58) Längsenden (64) aufweisen, die an dem Rahmen (20) koextensiv mit den dehnbaren Taillenbändern (11, 17) befestigt sind,
**dadurch gekennzeichnet, dass**
die Leckschutzvorrichtungen (58) in einer Querrichtung (4) entlang deren Längslänge elastomer sind.

2. Absorptionsfähiger Artikel (10) gemäß Anspruch 1, wobei der Artikel (10) das Trainingshöschen eines Kindes ist.

3. Absorptionsfähiger Artikel (10) gemäß Anspruch 1 oder 2, wobei der Rahmen (20) ein im Wesentlichen elastomeres äußeres Deckelement (30) beinhaltet, wobei die seitlichen Außenseiten der Leckschutzvorrichtungen (58) an dem elastomeren äußeren Deckelement (30) befestigt sind.

4. Absorptionsfähiger Artikel (10) gemäß Anspruch 1 oder 2, wobei der Rahmen (20) eine im Wesentlichen elastomere körperseitige Auskleidung (28) beinhaltet, wobei die seitlichen Außenseiten (62) der Leckschutzvorrichtungen (58) an der elastomeren körperseitigen Auskleidung (28) befestigt sind.

5. Absorptionsfähiger Artikel (10) gemäß Anspruch 1 oder 2, wobei der Rahmen (20) eine generell nicht elastomere Struktur umfasst, und des Weiteren elastomere Seitenbahnen (56) umfasst, die an seitlichen Seiten des Rahmens (20) befestigt sind, wobei die seitlichen Außenseiten (62) der Leckschutzvorrichtungen (58) an dem Rahmen (20) mit den elastomeren Seitenbahnen (56) befestigt sind.

6. Absorptionsfähiger Artikel (10) gemäß Anspruch 5, wobei die Seiten bahnen (56) seitlich sich erstreckende vordere und hinteren Bahnteile (50, 52) an gegenüberliegenden seitlichen Seiten des vorderen und hinteren Taillenbereichs (14, 12) definieren.

7. Absorptionsfähiger Artikel (10) gemäß Anspruch 6. wobei die Leckschutzvorrichtungen (58) Längsenden (64) aufweisen, die an den Seitenbahnen (56) in den Taillenbereichen (14, 12) des Rahmens (20) befestigt sind.

8. Absorptionsfähiger Artikel (10) gemäß einem der vorherigen Ansprüche, wobei die seitlichen Innenseiten (60) der Leckschutzvorrichtungen (58) in Längsrichtung elastisch sind.

9. Absorptionsfähiger Artikel (10) gemäß einem der vorherigen Ansprüche, wobei die Leckschutzvorrichtungen (58) aus einem flüssigkeitsundurchlässigen oder flüssigkeitsresistenten Neck-Bonding-Laminatmaterial gebildet sind.

10. Absorptionsfähiger Artikel (10) gemäß Anspruch 9, wobei das Neck-Bonding-Laminatmaterial eine Necked-Streck-Polypropylen-Spinnvliesbahn umfasst, die auf einen elastischen Film laminiert ist.

11. Absorptionsfähiger Artikel (10) gemäß einem der Ansprüche 1 bis 8, wobei die Leckschutzvorrichtungen (58) aus einem flüssigkeitsundurchlässigen Streck-Bonding-Laminatmaterial gebildet sind.

12. Absorptionsfähiger Artikel (10) gemäß Anspruch 1, wobei:
der Rahmen (20) einen generell elastomeren absorptionsfähigen Rahmen (20) umfasst, der eine körperseitige Auskleidung (28), eine äußere Deckschicht (30), die mit der körperseitigen Lage (28) verbunden ist und eine absorptionsfähige Struktur (32) umfasst, die zwischen der körperseitigen Auskleidung (28) und der äußeren Deckschicht (30) angeordnet ist;
wobei der vordere und hintere Taillenbereich (14, 12) des Weiteren sich seitlich erstreckende Seitenbahnenteile (56) umfasst, wobei die Seitenbahnenteile (56) Beinkonturen mit dem Schrittbereich (16) entlang gegenüberliegenden seitlichen Seiten des Rahmens (20) definieren;
wobei die Leckschutzvorrichtungen (58) die Auffangtaschen entlang der Beinkonturen und der Seitenbahnteile (56) definieren; und
beim Ausbilden des Rahmens (20) zu einer Hosenstruktur mit vorderen und hinteren Seitenbahnteilen (56), die an Seitensäumen (26) des Artikels (10) verbunden sind, die Leckschutzvorrichtungen (58) Leckschutz für einen Träger generell vollständig um den vorderen und hinteren Taillenbereich (14, 12) bereitstellen.

13. Absorptionsfähiger Artikel (10) gemäß Anspruch 12, wobei die absorptionsfähige Struktur (32) des Weiteren einen zentralen sich längs erstreckenden Teil und elastomere Flügelteile (32a) umfasst, die sich seitlich in den vorderen und hinteren Seitenbahnenteil (56) erstrecken.

14. Absorptionsfähiger Artikel (10) gemäß Anspruch 13, wobei die absorptionsfähige Struktur (32) ein elastomeres absorptionsfähiges Material entlang des zentralen Teils und der Flügelteile (32a) beinhaltet.

15. Absorptionsfähiger Artikel (10) gemäß Anspruch 14, wobei die absorptionsfähige Struktur (32) an der äußeren Deckschicht (30) entlang des zentralen Teils und der Flügelteile befestigt ist.

16. Absorptionsfähiger Artikel (10) gemäß einem der Ansprüche 12 bis 15, wobei sich die absorptionsfähige Struktur (32) zentral entlang des Schrittbereichs (16) zwischen dem vorderen und hinteren Taillenbereich (14, 12) erstreckt.

17. Absorptionsfähiger Artikel (10) gemäß einem der Ansprüche 12 bis 16, wobei der Artikel (10) ein Trainingshöschen eines Kindes ist.

18. Absorptionsfähiger Artikel (10) gemäß einem der Ansprüche 12 bis 17, wobei die Seitensäume (26) verbundene zerreißbare Säume sind.

19. Absorptionsfähiger Artikel (10) gemäß einem der Ansprüche 12 bis 17, wobei die Seitesäume (26) lösbar und wiederbefestigbar sind.

20. Absorptionsfähiger Artikel (10) gemäß einem der Ansprüche 12 bis 19, wobei die Leckschutzvorrichtungen (58) aus einem elastomeren Material gemacht sind.

## Revendications

1. Article absorbant (10), comprenant :
un châssis (20) comportant une région de ceinture avant (14) à une première extrémité longitudinale (15), une région de ceinture arrière (12) à une extrémité longitudinale opposée (13), et une région d'entrejambes (16) s'étendant longitudinalement entre lesdites région de ceinture avant et région de ceinture arrière (14, 12) ;
une structure de corps absorbante (32) s'étendant longitudinalement généralement à partir de ladite région de ceinture avant (14) jusqu'à ladite région de ceinture arrière (12) ; et
des protections contre les fuites s'étendant longitudinalement (58), chacune desdites protections contre les fuites (58) comportant un côté latéralement interne libre (60) et un côté latéralement externe (62) fixé audit châssis (20) de façon à définir des poches de confinement respectives le long de côtés latéraux opposés de ladite structure de corps absorbante (32), dans lequel ledit châssis (20) comprend des ceintures étirables (11, 17) le long d'au moins une partie desdites région de ceinture avant et région de ceinture arrière (14, 12), lesdites protections contre les fuites (58) comportant des extrémités longitudinales (64) fixées audit châssis (20) coextensives auxdites ceintures étirables (11, 17),
**caractérisé en ce que :**
lesdites protections contre les fuites (58) sont élastomères dans une direction transversale (4) le long de leur longueur longitudinale.

2. Article absorbant (10) selon la revendication 1, ledit article (10) étant une couche-culotte pour enfant.

3. Article absorbant (10) selon la revendication 1 ou 2, dans lequel ledit châssis (20) intègre un élément de revêtement externe fondamentalement élastomère (30), lesdits côtés latéralement externes desdites protections contre les fuites (58) étant fixés audit élément de revêtement externe élastomère (30).

4. Article absorbant (10) selon la revendication 1 ou 2, dans lequel ledit châssis (20) intègre une doublure fondamentalement élastomère destinée à se trouver du côté du corps (28), lesdits côtés latéralement externes (62) desdites protections contre les fuites (58) étant fixés à ladite doublure élastomère destinée à se trouver du côté du corps (28).

5. Article absorbant (10) selon la revendication 1 ou 2, dans lequel ledit châssis (20) comprend une structure généralement non élastomère, et comprenant en outre des panneaux latéraux élastomères (56) fixés à des côtés latéraux dudit châssis (20), lesdits côtés latéralement externes (62) desdites protections contre les fuites (58) étant fixés audit châssis (20) avec lesdits panneaux latéraux élastomères (56).

6. Article absorbant (10) selon la revendication 5, dans lequel lesdits panneaux latéraux (56) définissent des parties de panneaux avant et arrière s'étendant latéralement (50, 52) sur des côtés latéraux opposés desdites région de ceinture avant et région de ceinture arrière (14, 12).

7. Article absorbant (10) selon la revendication 6, dans lequel lesdites protections contre les fuites (58) comportent des extrémités longitudinales (64) fixées auxdits panneaux latéraux (56) au niveau desdites régions de ceinture (14, 12) dudit châssis (20).

8. Article absorbant (10) selon l'une quelconque des revendications précédentes, dans lequel lesdits côtés latéralement internes (60) desdites protections contre les fuites (58) sont élastiques dans la direction longitudinale (6).

9. Article absorbant (10) selon l'une quelconque des revendications précédentes, dans lequel lesdites protections contre les fuites (58) sont formées d'un matériau stratifié lié avec striction simultanée imperméable aux liquides ou résistant aux liquides.

10. Article absorbant (10) selon la revendication 9, dans lequel ledit matériau stratifié lié avec striction simultanée comprend une nappe filée fondue en polypropylène ayant subi une striction et un étirage appliquée par stratification sur un film élastique.

11. Article absorbant (10) selon l'une quelconque des revendications 1 à 8, dans lequel lesdites protections contre les fuites (58) sont formées d'un matériau stratifié lié avec étirage simultané imperméable aux liquides.

12. Article absorbant (10) selon la revendication 1, dans lequel :
ledit châssis (20) comprend un châssis absorbant généralement élastomère (20) comprenant une doublure destinée à se trouver du côté du corps (28), un revêtement externe (30) lié à ladite doublure destinée à se trouver du côté du corps (28), et une structure absorbante (32) disposée entre ladite doublure destinée à se trouver du côté du corps (28) et ledit revêtement externe (30) ;
lesdites région de ceinture avant et région de ceinture arrière (14, 12) comprennent en outre des parties de panneaux latéraux s'étendant latéralement (56), lesdites parties de panneaux (56) définissant des contours de jambes avec ladite région d'entrejambes (16) le long de côtés latéraux opposés dudit châssis (20) ;
lesdites protections contre les fuites (58) définissent lesdites poches de confinement le long desdits contours de jambes et desdites parties de panneaux latéraux (56) ; et
quand ledit châssis (24) est formé pour produire une structure de culotte, les parties de panneaux latéraux avant et arrière (56) étant jointes au niveau de joints latéraux (26) dudit article (10), lesdites protections contre les fuites (58) protègent une personne qui porte l'article contre les fuites généralement complètement autour des régions de ceinture avant et arrière (14, 12).

13. Article absorbant (10) selon la revendication 12, dans lequel ladite structure absorbante (32) comprend en outre une partie centrale s'étendant longitudinalement et des parties élastomères formant des ailes (32a) qui s'étendent latéralement dans lesdites parties de panneaux latéraux avant et arrière (56).

14. Article absorbant (10) selon la revendication 13, dans lequel ladite structure absorbante (32) intègre un matériau absorbant élastomère lie long de ladite partie centrale et desdites parties formant des ailes (32a).

15. Article absorbant (10) selon la revendication 14, dans lequel ladite structure absorbante (32) est fixée audit revêtement externe (30) le long de ladite partie centrale et desdites parties formant des ailes.

16. Article absorbant (10) selon l'une quelconque des revendications 12 à 15, dans lequel ladite structure absorbante (32) s'étend centralement le long de ladite région d'entrejambes (16) entre lesdites région de ceinture avant et région de ceinture arrière (14, 12).

17. Article absorbant (10) selon l'une quelconque des revendications 12 à 16, ledit article (10) étant une couche-culotte pour enfant.

18. Article absorbant (10) selon l'une quelconque des revendications 12 à 17, dans lequel lesdits joints latéraux (26) sont des joints déchirables collés.

19. Article absorbant (10) selon l'une quelconque des revendications 12 à 17, dans lequel lesdits joints latéraux (26) sont libérables et réattachables.

20. Article absorbant (10) selon l'une quelconque des revendications 12 à 19, dans lequel lesdites protections contre les fuites (58) se composent d'un matériau élastomère.
